# EUROPÄISCHE PATENTSCHRIFT

(11) **EP 2 364 683 B1**
(45) Veröffentlichungstag und Bekanntmachung des Hinweises auf die Patenterteilung: **15.07.2015**
(21) Anmeldenummer: 11155223.8
(22) Anmeldetag: 21.02.2011
(51) Int. Cl.: A61F 13/08

(54) **Kompressionsbandage und Verfahren zu deren Herstellung**
Compression bandage and method for producing same
Bandage de compression et son procédé de fabrication

(30) Priorität: 11.03.2010 DE 102010011840
(43) Veröffentlichungstag der Anmeldung: 14.09.2011
(73) Patentinhaber: Peter Müller GmbH, 72461 Albstadt (DE)
(72) Erfinder: Müller, Markus, 72461, Albstadt (DE)
(74) Vertreter: Thum, Bernhard

(56) Entgegenhaltungen:
- CH-A- 103 772
- DE-C- 860 531
- DE-U1- 8 702 573
- US-A- 2 280 025

## Beschreibung

### Anwendungsgebiet und Stand der Technik

Die Erfindung betrifft eine Kompressionsbandage zum Umgeben eine Körperteils mit einem röhrenförmigen flexibler Flächengebilde zum Umgeben des Körperteils und mit einer Mehrzahl elastischer Kompressionfäden. Das Flächengebilde bildet einen in Umfangsrichtung streckbaren Hauptkörper der Kompressionsbandage. Die Kompressionsfäden verlaufen in der Ebene des Flächengebildes in Umfangsrichtung durch das Flächengebilde geführt und erzielen im angelegten Zustand der Kompressionsbandage die beabsichtigte Kompressionswirkung. Die Erfindung betrifft weiterhin ein Verfahren zur Herstellung einer Kompressionsbandage.

Gattungsgemäße Kompressionsbandagen sind aus dem Stand der Technik bekannt, wie z.B aus der DE 8702573 U. Sie dienen der Kompression eines Körperteils eines Patienten, insbesondere eines Arms oder eines Beins, zu therapeutischen Zwecken. Gattungsgemäße Kompressionsbandagen werden insbesondere im Rahmen einer Therapie von Ödemen wie beispielsweise Lymphödemen verwendet.

Das röhrenförmige Flächengebilde, welches den Hauptkörper der Kompressionsbandage bildet, wird über das zu behandelnde Körperteil gezogen, wobei die im Hauptkörper enthaltenen elastischen Kompressionsfäden, die sich im angelegten Zustand um das Körperteil herum erstrecken, beim Anlegen gedehnt werden und dadurch die gewünschte Kompressionswirkung erziele. Gattungsgemäße Kompressionsbandagen sind in verschiedenen Kompressionsklassen erhältlich, wobei üblicherweise die Klassen I bis IV unterschieden werden, die sich durch das Maß des bewirkten Kompressionsdrucks unterscheiden.

Auch wenn im Rahmen einer therapeutischen Anwendung einer gattungsgemäßen Kompressionsbandage vorgesehen ist, dass über die Therapiedauer hinweg ein gleichbleibender Kompressionsdruck erzielt werden soll, führt dies bislang dennoch zu dem Erfordernis, in verschiedenen Stadien der Therapie verschiedene Kompressionsbandagen zu verwenden, da die gewünschte therapeutische Wirkung, insbesondere beim Vorhandensein von Ödemen, mit sich bringen kann, dass das zu behandelnde Köperteil an Umfang verliert Die Beibehaltung der gleichen Kompressionsbandage würde damit zu einer immer geringer werdenden Kompressionswirkung führen.

Diesem Problem wird üblicherweise dadurch begegnet, dass dem Patienten mit fortschreitender Therapie neue Kompressionsbandagen angepasst werden, die hierfür aufwendig individuell hergestellt werden müssen. Ein Umarbeiten bestehender Kompressionsbandagen ist dabei in der Regel nicht möglich, da die hierfür erforderliche Auftrennung der nicht mehr angemessenen Bandage dazu führen würde, dass die Kompressionsfäden ihren Halt verlieren und ihre Wirkung daher nicht mehr erzielen können. So kommt beispielsweise insbesondere ein Öffnen des röhrenförmigen Hauptkörpers und ein nachfolgendes Abtrennen eines Randbereichs des Flächengebildes des Hauptkörpers zur Verringerung des Umfangs nicht in Betracht, da in oben skizzierter Weise dadurch die Kompressionsfäden, die im abgetrennten Randbereich fixiert waren, nicht mehr beidendig gehalten wurden und somit Ihre Wirkung nicht mehr entfalten könnten.

Aus dem Stand der Technik ist es bekannt, eine Kompressionsbandage offen zu gestalten. In einem solchen Fall wird die röhrenförmige Gestalt dadurch hergestellt, dass in einem Lieferzustand voneinander getrennte und sich gegenüberliegende Randbereiche der Bandagenfläche zur Anpassung an spezifische Anforderungen miteinander durch einen Klettverschluss verbunden werden. Hierdurch lässt sich eine gewünschte Kompressionswirkung allerdings nur sehr ungenau einstellen. Außerdem ist ein solcher Klettverschluss für den Patienten einfach handhabbar, so dass die Gefahr besteht, dass der Patient durch Veränderung des Umfangs der Kompressionsbandage die zuvor eingestellte Konfiguration und damit die gewünschte Kompressionswirkung verändert.

### Aufgabe und Lösung

Aufgabe der Erfindung ist es, eine gattungsgemäße Kompressionsbandage zur Verfügung zu stellen, die die Nachteile des Standes der Technik vermindert. Erfindungsgemäß wird dies dadurch erzielt, dass bei einer gattungemäßen Kompressionsbandage zumindest einige der Kompressionsfäden zumindest abschnittsweise aus dem Hächengebilde herausragen, so dass sie eine Einstellbarkeit der Kompressionswirkung durch Variierung der Länge ihres jeweils herausragenden Außenabschnitts gewährleisten.

Eine erfindungsgemäße Kompressionsbandage weist somit den bereits beschriebenen röhrenförmigen Hauptkörper auf, in dem die Kompresse onsfäden verlaufen. Dieser röhrenförmige Hauptkörper ist geschlossen ausgebildet und soll bestimmungsgemäß, weder durch das die Bandage verschreibende medizinisch Personal noch durch den Patienten selbst bezüglich seines Umfangs geöffnet werden. Zur individuellen Anpassung der Kompressionswirkung sind zumindest einige der in Umfangs" richtung verlaufenden Kompressionsfäden aus dem Hauptkörper nach außen herausgeführt, so dass durch Verändern eines Anteils des Kompressionsfadens, der den Außenabschnitt bildet, die Kompressionswirkung des Kompressionsfadens gezielt eingestellt werden kann. Insbesondere kann auch ein Nachstellen einer bereits im Rahmen einer Therapie verwendeten Kompressionsbandage erfolgen, so dass diese auch nach der im Rahmen der Therapie bereits eingetretenen und gewünschten Verringerung des Umfangs des Körperteils noch eine gleichbleibende Kompressionswirkung erzielen kann.

Die Kompressionsfäden umgeben das Körperteil zumindest größtenteils. Bei der Verwendung eines Rundgestricks als Hauptkörper ist auch ein vollständiges Umgeben des Körperteils möglich. Zumindest jedoch wird ein Umgeben des Körperteils von 75%, d.h. ein Umschlingungswinkel von 270°, als bevorzugt angesehen. Die erfindungsgemäße Kompressionsbandage verfügt über eine Vielzahl von Kompressionsfäden, die in Umfangsrichtung und im Wesentlichen parallel zueinander verlaufen. Im Sinne dieser Erfindung werden dabei auch zueinander parallel verlaufende Teile eines gemeinsamen Fadens, der beispielsweise als Sehussfaden mit um 180° wechselnden Orientierungen mehrfach versetzt zueinander durch den Hauptkörper geführt ist, als separate Kompressionsfäden angesehen. Die Kompressionsfäden können bei der Verwendung eines textilen Flächengebildes für den Hauptkörper insbesondere durch dessen Maschen geführt sein, so dass das Vorsehen davon separater Führungskanäle nicht erforderlich ist.

Die Kompressionsfäden bestehen vorzugsweise aus Gummi, insbesondere Naturkautschuk. Auch ein künstliches Material ist wie Elastan kann verwendet werden. Zur Erzielung eines gewünschten Reibungsverhaltens mit dem im Hauptkörper verarbeiteten Garn kann es zweckmäßig sein, dass die Kompressionsfäden eine eine Seele aus Gummi oder Elastan umgebende Schicht aus konventionellem Garn aufweisen. Abhängig von der konkreten Kompressionsbandage kann sowohl die Erzielung eines sehr geringen Reibungskoeffizienten als auch die Erzielung eines hohen Reibungskoeffizienten von Vorteil sein. Eine geringe Reibung erleichtert die Relativbewegung der Kompressionsfäden gegenüber dem Hauptkörper, so dass sich über den Umfang der Kompressionsbandage ein weitgehend homogener Kompressionszustand einstellt. Durch eine hohe Reibung wird dagegen die Fixierung eines durch Variieren der Länge des Außenabschnitts eingestellte Kompressionswirkung unterstützt.

Die herausragenden Außenabschnitte der Kompressionsfäden sind durch ein Festlegungsmittel so zu fixieren, dass eine durch eine fachkundige Person eingestellte Konfiguration erhalten bleibt.

Der Hauptkörper, der durch das flexible Flächengebilde gebildet wird, ist vorzugsweise als textiler Hauptkörper ausgebildet. Wie bereits erwähnt, kann er dabei als Rundgestrick vorgesehen sein. Als vorteilhaft wird es jedoch angesehen, wenn das Flächengebilde durch Verbinden gegenüberliegender Randbereiche eines Flachgebildes hergestellt ist. In einem solchen Fall wird ein Flachgebilde verwendet, insbesondere ein Flachgestrick, bei dem gegenüberliegende Kanten durch eine Naht o.ä. miteinander verbunden werden, um dadurch den röhrenförmigen Hauptkörper zu bilden. Diese Herstellungsmethode erlaubt es insbesondere auf einfache Weise, durch zwischen den Maschenreihen variierende Maschenzahl von der Zylinderform abweichende Hauptkörper zu bilden, wie es beispielsweise im Falle einer Beinbandage oder eines Stützstrumpfes erforderlich ist.

Bei der Verwendung eines solchen Flachgebildes, insbesondere eines Flachgestricks, ist es insbesondere von Vorteil, wenn die Kompressionsfäden zumindest teilweise Teil eines gemeinsamen Fadens sind, der mehrfach zwischen den gegenüberliegenden Randbereichen des Flächengebildes hin- und herführt. Hierdurch wird die Fertigung vereinfacht, da die elastischen Kompressionsfäden als Schussfäden während der Herstellung des Flachgestricks eingebracht werden können.

Von besonderem Vorteil ist es dabei, wenn Austrittstellen, an denen die Außenabschnitte der Kompressionsfäden aus dem Flächengebilde herausragen, von dem zugeordneten Rand beabstandet sind, vorzugsweise um mindestens fünf Millimeter, insbesondere um mindestens 10 Millimeter. Die Kompressionsfäden, die sich im Falle eines Flachgestricks zwischen zwei miteinander zu vernähenden Randbereichen erstrecken, können durch diese vom Rand beabstandeten Austrittsstellen vom Bereich der Naht zwischen den Rändern ferngehalten werden, so dass ihre Beweglichkeit an den Austrittsstellen nicht durch die Naht verschlechtert wird. Von besonderem Vorteil ist es, wenn die jenseits dieser Austrittstellen und daher von Kompressionsfäden freien Bereiche des Flachgebildes gegenüber dem restlichen Hauptkörper verstärkt und/oder versteift sind, so dass trotz des Fehlens der Kompressionsfäden in diesem Bereich ein Faltenwurf an dieser Stelle vermieden wird.

Zum Zwecke der bequemen Einstellbarkeit der jeweiligen Länge der Außenabschnitte der Kompressionsfäden ist es bevorzugt, wenn eine Mehrzahl von aus dem Flächengebilde herausragenden Kompressionsfäden mit einem gemeinsamen Handhabungsabschnitt verbunden ist, mittels dessen diese Kompressionsfäden aus dem Flächengebilde partiell herausgezogen werden können. Dieser Handhabungsabschnitt gestattet es somit, mehrere oder alle Kompressionsfäden gleichzeitig zu verstellen.

Auch ist es möglich, eine Mehrzahl von Handhabungsabschnitten vorzusehen, mit der jeweils eine Mehrzahl von Kompressionsfäden verbunden ist, so dass durch die unterschiedlichen Handhabungsabschnitte in einzelnen Teilbereichen der Kompressionsbandage jeweils für einzelne Körperbereiche ein gewünschter Kompressionszustand spezifisch einstellbar ist.

Der oder die Handhabungsabschnitte können als weitgehend starre Bauteile, beispielsweise als einstückige Kunststoffabschnitte ausgebildet sein.

Von besonderem Vorteil ist es jedoch, wenn der Handhabungsabschnitt bzw. die Handhabungsabschnitte flexibel ausgebildet ist, so dass die an verschiedenen Stellen mit diesem formverändetlichen Handhabungsabschnitt verbundenen Kompressionsfäden in unterschiedlichem Maße aus dem Hauptkörper herausgezogen werden können, um dadurch bereichsspezifisch eine Kompressionswirkung einstellen zu können.

Hierbei ist es insbesondere von Vorteil, wenn der Handhabungsabschnitt als mit dem Flächengebilde nur durch die Kompressionsfäden verbundener textiler Abschnitt ausgebildet ist, wobei dessen Maschen die Kompressionsfäden führen und stützen.

Die Gestaltung mit einem textilen Handhabungsabschnitt bietet neben haptischen Vorteilen auch einen wesentlichen Vorteil hinsichtlich der Herstellung der Kompressionsbandage, da der Hauptkörper bzw. das den Hauptkörper bildende Flachgestrick und der Handhabungsabschnitt bzw. dessen Flachgestrick in einem gemeinsamen Verfahrensschritt hergestellt werden können. Dies wird im Weiteren noch erläutert.

Wie oben bereits erwähnt, bedarf es eines Festtegungsmitte!s um einen Zustand fixieren zu können, der zuvor durch therapeutisch zweckmäßiges Herausziehen der Koimpressionsfäden aus dem Hauptkörper erreicht wurde. Vorzugsweise ist vorgesehen, dass der Handhabungsab-schnitt selbst an einer Außenseite des Hauptkörpers durch dieses Festlegungsmittel festlegbar bzw. festgelegt ist. Das Festlegutigsmittel kann dabei beispielsweise als Klettverschluss oder durch Haken gebildet sein, die in das Gestrick des Hauptkörpers eingehängt werden. Von Vorteil ist es jedoch, wenn das Festlegungsmittel derart ausgebildet ist, dass es durch einen Patienten nicht werkzeuglos lösbar ist. Hierdurch wird verhindert, dass der Patient willentlich oder unwillentlich eine durch fachkundiges Personal eingestellte Konfiguration verändert. Das Festlegungsmittel kann beispielsweise derart ausgebildet sein, dass es nur mittels eines speziellen Werkzeugs, beispielsweise eines Nietöffners oder einer Wärmequelle lösbar ist. Bevorzugte Beispiele für ein solches für den Patienten nicht ohne weiteres lösbares Festlegungsmittel sind eine Naht, eine Nietenverbindung, eine Schweißverbindung und eine Klebeverbindung.

Im Falle der Verwendung einer Nietenverbindung ist bevorzugt, wenn im Hauptkörper und/oder am Handhabungsabschnitt bereits Ausnehmungen vorgesehen sind, die zur Aufnahme der Nieten dienen. Im Falle der Verwendung einer Naht ist es bevorzugt, wenn ein Nahttyp Verwendung findet, der bei lokaler Beschädigung im Übrigen seine Stabilität bewahrt, beispielsweise durch Verknotungen des nahtbildenden Garns. So wird verhindert, dass eine versehentliche Beschädigung der Naht unmittelbar zu einem Verlust der eingestellten Kompressionswirkung führt.

Eine erfindungsgemäße Kompressionsbandage kann vorzugsweise als Armbandage, als Beinbandage oder als Kompressionsstrumpf ausgebildet sein. Im Falle einer Armbandage weist eine erfindungsgemäße Kompressionsbandage vorzugsweise eine Länge von bis zum 30 cm auf. Als Beinbandage weist eine erfindungsgemäße Kompressionsbandage vorzugsweise eine Länge von mehr als 40 cm auf. Kompressionsstrümpfe, können eine Länge bis 105 cm aufweisen. Sie können zehen" seitig offen oder geschlossen ausgebildet sein.

Insbesondere bei Beinbandagen kann es vorteilhaft sein, die Kompressionsfäden derart zu führen, dass spannungsreduzierte Zonen entstehen, beispielsweise im Bereich der Kniescheibe. Diese spannungsreduzierten Zonen können durch Einarbeiten eines starren Einsatzes in den Hauptkörper gebildet sein.

Die Herstellung einer erfindungsgemäßen Kompressionsbandage kann derart erfolgen, dass die Bandage in an sich üblicher Weise hergestellt wird und anschließend die im Rundgestrick oder kantenseitig verbundenen Flachgestrick enthaltenen Kompressionsfäden entlang einer in Haupterstreckungsrichtung der Bandage verlaufenden Linie einzeln aus dem Gestrick herausgezogen werden, um dann beispielsweise mit einem gemeinsamen Handhabungsabschnitt verbunden zu werden.

Von besonderem Vorteil ist jedoch, ein Herstellungsverfahren zur Herstellung einer erfindungsgemäßen Kompressionsbandage, bei dem das flexible Flächengebilde als Flachgestrick auf einer Strickmaschine hergestellt wird, wobei während des Strickvorganges die Kompressionsfäden in das Flachgestrick eingebracht werden und wobei ein Außenabschnitt zumindest eines Teils der Kompressionsfäden während des Strickvorganges aus dem flexiblen Flächengebilde herausgeführt wird. Anschließens werden zwei gegenüberliegende Kanten des Flachgestricks miteinander verbunden, um dadurch den röhrenförmigen Hauptkörper zu bilden.

Die während des Strickvorganges herausgeführten Außenabschnitte können dann zur oben beschriebenen Einstellung der Kompressionsbandage genutzt werden. Sie weisen eine außenliegende Länge von vorzugsweise mindestens 10 Millimetern auf. Dies erlaubt die anschließende Verbindung mit einem Handhabungsabschnitt. Vorzugsweise werden die Kompressionsfäden beabstandet von den zu verbindenden Kanten aus dem Flachgestrick herausgeführt.

Von besonderem Vorteil ist eine Variante des Herstellungsverfahrens, bei dem die Strickmaschine zeitgleich und parallel zur Herstellung des flexiblen Flächengebildes des Hauptkörpers einen ebenfalls als Flachgestrick ausgebildeten Handhabungsabschnitt herstellt, wobei freie Enden der Außenabschnitte der Kompressionsfäden in dieses Flachgestrick des Handhabungsabschnittes eingelegt und dadurch fixiert werden.

Bei dieser Ausgestaltung ist demnach vorgesehen, dass die Strickmaschine zeitgleich sowohl die Maschenreihen des späteren Hauptkörpers als auch die Maschenreihen des Handhabungsabschnittes herstellt. Dabei werden die Kompressionsfaden, vorzugsweise in Form eines durchgehenden Schussfadens, vorn späteren Hauptkörper zum Handhabungsabschnitt und gegebenenfalls wieder zurückgeführt. Ergebnis dieser Herstellungsmethode sind zwei Flachgestricke, die den späteren Hauptkörper und den Handhabungsabschnitt bilden und die miteinander lediglich über die Kompressionsfäden verbunden sind. Die Kompressionsfäden erstrecken sich somit über eine Lücke, die zwischen den beiden Flachgestricken verbleibt.

Vorteil dieser Methode ist neben der Tatsache, dass somit die Herstellung einer erfindungsgemäßen Bandage in nur wenigen Schritten möglich ist, die Tatsache, dass die Außenabschnitte der herausgeführten Kompressionsfäden nicht drohen, noch vor Anbringung eines Handhabungsabschnittes Knoten zu bilden oder sich anderweitig zu verwirren.

### Kurzbeschreibung der Zeichnungen

Weitere Aspekte und Vorteile der Erfindung ergeben sich aus der nachfolgenden Beschreibung eines bevorzugten Ausführungsbeispiels der Erfindung, welches anhand der Figuren erläutert wird. Dabei zeigen:
- Fig. 1 und 1a: ein Halbzeug zur Herstellung einer erfindungsgemäßen Kompressionsbandage und
- Fig. 2a bis 2c: die Einstellbarkeit der Kompressionsbandage.

### Detaillierte Beschreibung des Ausführungsbeispiels

Das nachfolgend erläuterte Ausführungsbeispiel bezieht sich auf eine im Kniebereich eines Patienten zu tragende Kompressionsbandage für ein Bein. Diese Art der Kompressionsbandage ist lediglich beispielhaft zu verstehen. Die nachfolgend beschriebene Art der Hersteillung und der Konfiguration der Kompressionsbandage gilt gleichermaßen auch für anderweitige Kompressionsbandagen, beispielsweise in Form von Kompressionsstrümpfen oder Armbandagen.

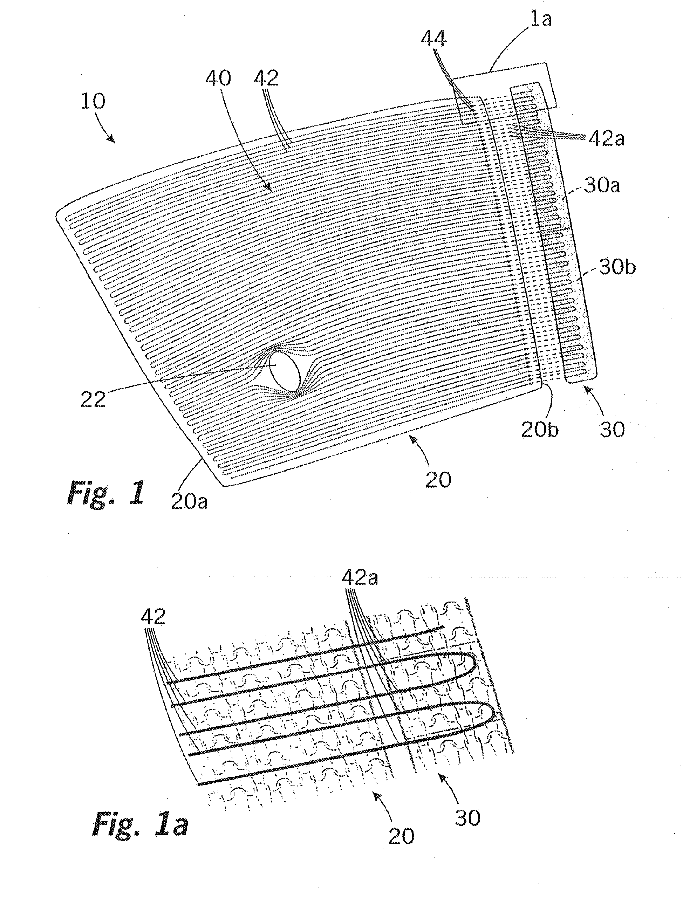

Die Fig. 1 zeigt die erfindungsgemäße Kompressionsbandage im noch nicht fertig hergestellten Zustand.

In diesem Zustand besteht die spätere Kompressionsbandage '10 aus einem den späteren Hauptkörper 50 bildenden Flachgestrick 20 und einem einen späteren Handhabungsabschnitt 60 bildenden schmalen Flachgestrickstreifen 30. Die Flachgestricke 20, 30 bestehen naturgemäß in der in Fig. 1a dargestellten Weise aus einzelnen Maschen, wobei es sich bei dem verwendeten Garn um das weitgehend unelastische Polyamid 6.6 handeln kann. Dieses kann gegebenenfalls noch mit einem elastischen Elastanfaden plattiert sein.

Zur Anlage im späteren Kniebereich weist das Flachgestrick 20 einen darin eingearbeiteten in etwa elliptischen Kunststoffeinsatz 22 auf, der dazu dient, den späteren Kniebereich weitgehend spannungsfrei zu halten.

Bereits während der Herstellung der Flachgestricke 20, 30 ist ein aus Naturkautschuk bestehender Gummifaden 40 als Schussfaden eingebracht worden, dessen zueinander weitgehend parallele Abschnitte 42 im Sinne dieser Erfindung als Kompressionsfäden 42 bezeichnet werden. Diese Kompressionsfäden 42 erstrecken sich vom bezogen auf Fig. 1 linksseitigen Rand 20a des Hachgestricks 20 bis fast zu dessen rechtsseitigem Rand 20b. Etwa 10 mm vor dem rechtsseitigen Rand 20b verlassen die Kompressionsfäden 42 das Flachgestrick 20 an dessen Vorderseite durch Austrittstellen 44. Diese Austrittsstelleii 44 sind in den Figuren jeweils als kreisförmige Öffnungen dargestellt. Vorzugsweise handelt es sich jedoch lediglich um Zwischenbereiche zwischen Maschen, die dementsprechend keine kreisförmige Form aufweisen.

Jenseits der Austrittstellen 44 bilden die Kompressionsfäden Außenabschnitte 42a die zum streifenförmigen Flachgestrick 30 geführt: sind, welches später den Handhabungsabschnitt 60 bildet. Dort werden die Außenabschnitte durch die Maschen des Flachgestricks 30 gehalten.

Die Herstellung der beiden Flachgestricke 20, 30 der Fig. 1 erfolgt durch eine Strickmaschine, die beide Flachgestricke 20, 30 zeitgleich herstellt und dabei die in Fig. 1a dargestellten Maschenreihen beider Flachgestricke 20, 30 bildet, bevor jeweils die nächste Maschenreihe der Flachgestricke 20, 30 anschließend gebildet wird. Der Gummifaden 40, der die Kompressionsfäden 42 bildet, wird während der Herstellung der Maschenreihen jeweils als Schussfaden eingebracht. Es bedarf somit keines separaten Einbringens der Kompressionsfäden 42. Stattdessen werden diese in der insbesondere in Fig. 1a ersichtlichen Weise während der Herstellung der Flachgestricke 20, 30 in die Maschen eingeführt und somit fixiert.

Zur Herstellung der fertigen Kompressionsbandage 10 bedarf es augehend vom Zustand der Fig. 1 nur noch des Verbindens der gegenüberliegenden Ränder 20a, 20b des Flachgestricks 20. Das Flachgestrick 20 wird hierdurch in eine röhrenförmige Form gebracht, die den Hauptkörper 50 der Kompressionsbandage 10 bildet.

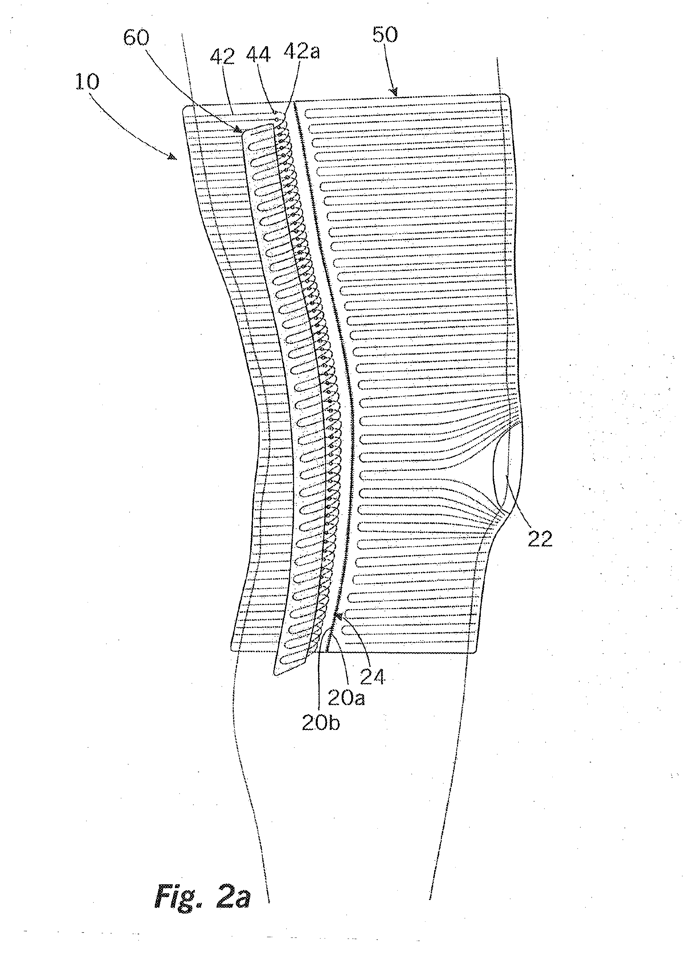

Die Fig. 2a zeigt die fertige Kompressionsbandage 10 im angelegten Zustand. Es ist ersichtlich, dass das Flachgestrick 20 im Bereich seiner Ränder 20a, 20b mittels einer Naht 24 verbunden ist und dadurch den röhrenförmigen Hauptabschnitt 50 bildet. Die Kompressionsfäden 42 reichen von beiden Seiten der Naht 24 bis fast an diese heran. Bezogen auf die Perspektive der Fig. 2a sind sie linksseitig der Naht 24 durch die bereits beschriebenen Austrittsstellen 44 herausgeführt und dann in der bereits beschriebenen Weise mit dem Handhabungsabschnitt 60 verbunden, der durch das in Fig. 1 dargestellte Flachgestrick 30 gebildet wird.

Fig. 2a zeigt den Zustand, in dem die Kompressionsbandage bereits angelegt ist, jedoch noch nicht auf die individuellen therapeutischen Belange des Patienten eingestellt ist. Dies erfolgt nach dem Anlegen in der durch die Fig. 2a bis 2c verdeutlichten Weise.

Abhängig von der jeweils herzustellenden Kompressionswirkung werden mittels des Handhabungsabschnittes 60 die Kompressiotisfäden 42 in gleichem oder in verschiedenem Maße aus dem Hauptkörper 50 herausgezogen, so dass der Anteil der Kompressionsfäden 42, der im Hauptkörper 50 verbleibt, eine Veränderung erfährt. Je größer der Außenabschnitt 42a der jeweiligen Kompressionsfäden 42 ist, desto stärker ist die bewirkte Kompression.

Zur Fixierung eines eingestellten Zustandes wird der Handhabungsabschnitt 60 mit einem Festlegungsmittel 70 am Hauptkörper 50 befestigt. Dieses Festlegungsimittel kann beispielsweise durch einen nicht dargestellten Klettverschluss gebildet sein, dessen komplementären Flächen einerseits an der innenseite des Handhabungsabschnittes 60 und andererseits an der Außenseite des Hauptabschnittes 50 vorgesehen sind.

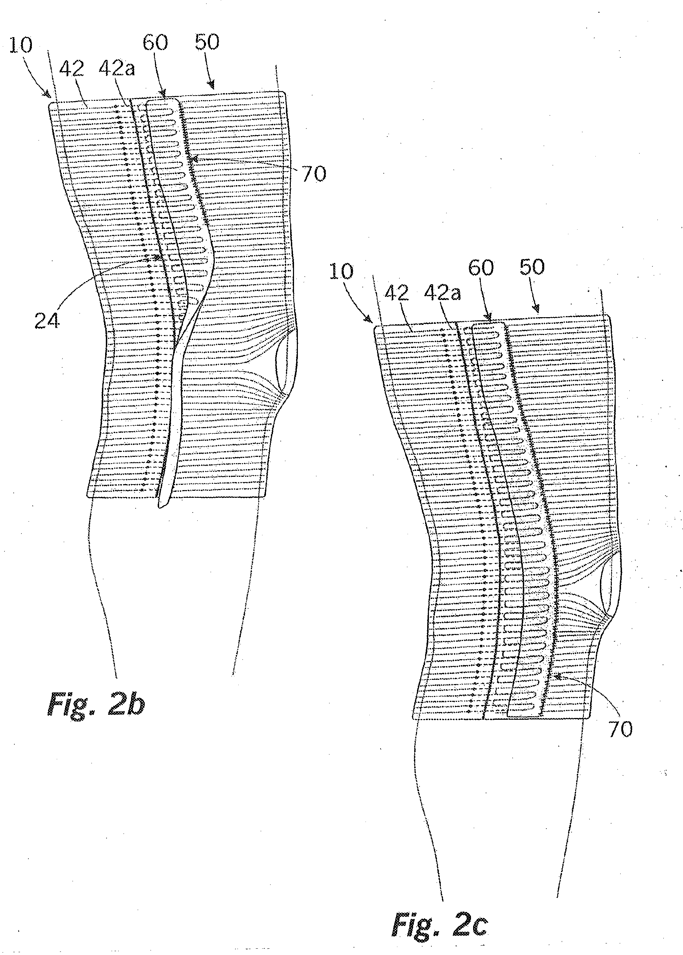

Bei dem dargestellten Ausführungsbeispiel wird das Festiegungsmittel allerdings primär durch eine Naht 70 gebildet, die in den Fig. 2b und 2c dargestellt ist. Mittels dieser Naht 70 wird der Handhabungsabschnitt 60 an der Außenfläche des Hauptabschnittes 50 befestigt, so dass die zuvor hergestellte Einstellung hinsichtlich der Kompressionsfäden 42 erhaben bleibt, Wie sich aus den Darstellungen der Fig. 2b und 2c ergibt, kann dabei insbesondere sukzessive eine Festlegung des Handhabungsabschnittes 60 am Hauptkörper 50 erfolgen, so dass die Herstellung der Naht 70 bereits begonnen wird, noch bevor anderenorts die gewünschte Kompressionswirkung eingestellt wurde. So kann durch fachkundiges Personal eine jeweils bereichsspezifisch geeignete Kompressionswirkung bequem hergestellt werden,

Die Fig. 2c zeigt die Kompressionsbandage nach Fertigstellung der Naht 70. Der fixierte Kompressionszustand bleibt auch nach Ablegen der Kompressionsbandage erhalten.

Sobald der eintretende Therapieerfolg eine Veränderung der Einstellung erfordert, kann die Naht 70 durch fachkundiges Personal aufgetrennt werden, um nach Anpassung der Länge der Außenabschnitte 42a bezogen auf den Hauptkörper 50 an anderer Stelle neu hergestellt zu werden und somit den veränderten therapeutischen Bedürfnissen des Patienten Rechnungzutragen.

Die Naht 70 als hier verwendetes Festlegungsmittel kann auch durch einen Klettverschluss ergänzt werden. Dies gestattet es, mittels des Klettverschlusses eine vorläufige Fixierung zu erzielen, die anschließend durch die Naht 70 oder anderweitige durch den Patienten nicht veränderbare Festlegungsmittel dauerhaft - also bis zur nächsten Korrektur durch fachkundiges Personal - fixiert zu werden.

Beim dargestellten Ausführungsbeispiel wird die bereichsspezfische Einstellbarkeit der Länge der Außenabschnitte 42 dadurch möglich, dass der Handhabungsabschnitt 60 formflexibel ausgebildet ist. Alternativ oder zusätzlich kann zum gleichen Zwecke auch eine Gestaltung mit mehreren Handhabungsabschnitten vorgesehen sein. Durch die gepunkteten Trennlinien am Flachgestrick 30 ist Fig. 1 ist Möglichkeit der Trennung des einen Handhabungsabschnitts in zwei separate Flachgestricke 30a, 30b angedeutet, die später separate Handhabungsabschnitte bilden.

Das Ausführungsbeispiel der Fig. 2a bis 2c zeigt die Einstellung der Kompressionsbandage im angelegten Zustand. Dieses Vorgehen ist nicht zwingend erforderlich. Die Festlegung des Handhabungsabschnittes 60 am Hauptkörper 50 kann unter Rückgriff auf Erfahrungswerte des fachkundigen Personals auch im nicht angelegten Zustand erfolgen.

## Patentansprüche

1. Kompressionsbandage (10) zum Umgeben eines Körperteils mit
- einem röhrenförmige flexiblen Flächengebilde (50) zum Umgeben des Körperteils, wobei dieses Flächengebilde (50) einen in Umfangsrichtung streckbaren Hauptkörper (50) bildet, und
- mit einer Mehrzahl) elastischer Kompressionsfäden (42), die in der Ebene des Flächengebildes (50) in Umfangsrichtung durch das Flächengebilde geführt verlaufen und im angelegten Zustand der Kompressionsbandage (10) die Kompressionswirkung erzielten,
**dadurch gekennzeichnet, dass**
zumindest einige der Kompressionsfäden (42) zumindest abschnittsweise aus dem Flächengebilde (50) herausragen, so dass sie eine Einstellbarkeit der Kompressionswirkung durch Variierung der Länge ihres jeweils herausragenden Außenabschnitts (42a) gewährleisten.

2. Kompressionsbandage (10) nach Anspruch 1,
**dadurch gekennzeichnet, dass**
das röhrenförmige Flächengebilde (50) durch Verbinden gegenüberliegender Randbereiche (20a, 20b) eines Flachgebildes (20) hergestellt ist.

3. Kompressionsbandage (10) nach Anspruch 2,
**dadurch gekennzeichnet, dass**
die Kompressionsfäden (42) zumindest teilweise Teil eines gemeinsamen Fadens (40) sind, der mehrfach zwischen den Randbereichen (20a, 20b) des Flächengebildes hin- und herläuft.

4. Kompressionsbandage (10) nach Anspruch 2 oder 3,
**dadurch gekennzeichnet, dass**
Austrittsstellen (44), an denen die Außenabschnitt der Kompressionsfäden (42) aus dem Flächengebilde herausragen, von dem zugeordneten Rand beabstandet sind, vorzugsweise um mindestens 5 mn, insbesondere vorzugsweise um mindestens 10mm.

5. Kompressionsbandage (10) nach einem der vorstehenden Ansprüche,
**dadurch gekennzeichnet, dass**
eine Mehrzahl von aus dem Flächengebilde (50) herausragenden Kompressionsfäden (42) mit einem gemeinsamen Handhabungsabschnitt (60) verbunden ist, mittels dessen die Kompressionsfäden (42) aus dem Flächengebilde (50) herausgezogen werden können.

6. Kompressionsbandage nach Anspruch 5,
**dadurch gekennzeichnet, dass**
eine Mehrzahl von Handhabungsabschnitten vorgesehen ist, mit der jeweils eine Mehrzahl von Kompressionsfäden verbunden ist, so dass durch die verschiedenen Handhabungsabschnitte in einzelnen Teilbereichen der Kompressionsbandage jeweils individuell ein gewünschter Kompressionszustand einstellbar ist.

7. Kompressionsbandage (10) nach Anspruch 5 oder 6,
**dadurch gekennzeichnet, dass**
das Flächengebilde (50) als textiles Flächengebilde ausgebildet ist, dessen Maschen die Kompressionsfäden (42) führen, wobei vorzugsweise auch der Handhabungsabschnitt (60) als mit dem Flächengebilde (50) nur durch die Kompressionsfäden (42) verbundener textiler Abschnitt ausgebildet ist, dessen Maschen ebenfalls die Kompressionsfäden (42) führen.

8. Kompressionsbandage (10) nach einem der Ansprüche 5 bis 7,
**dadurch gekennzeichnet, dass**
der Handhabungsabschnitt (60) an einer Außenweite des Hauptkörpers (50) durch ein Festlegungsmittel (70) festlegbar oder festgelegt ist, wobei das Festlegungsmittel (70) vorzugsweise derart ausgebildet ist, dass es durch einen Patienten nicht werkzeuglos lösbar ist, und/oder wobei das Festlegungsmittel (70) vorzugsweise
- eine Naht (70),
- eine Nietenverbindung,
- Schweißverbindung oder
- eine Klebeverbindung umfasst,

9. Kompressionsbandage (10) nach einem der vorstehenden Ansprüche,
**dadurch gekennzeichnet, dass**
die Kompressionsbandage
- als Armbandage
- als Beinbandage (10) oder
- Kompressionsstrumpf ausgebildet ist.

10. Verfahren zur Erstellung einer Kompressionsbandage (10) nach einem der vorstehenden Ansprüche,
**dadurch gekennzeichnet, dass**
1. das flexible Flächengebilde (50) als Flachgestrick (20) auf einer Strickmaschine hergestellt wird, wobei während des Strickvorgangs die Kompressionsfäden (42) in das Flachgestrick eingebracht werden und wobei ein Außenabschnitt (42a) zumindest eines Teils der Kompressionsfäden (42) während des Strickvorgangs aus dem flexiblen Flächengebilde (20) herausgeführt wird, und
2. zwei gegenüberliegende Kanten (20a, 20b) des Flachgestricks (20) miteinander verbunden werden, um den röhrenförmigen Hauptkörper (50) zu bilden.

11. Verfahren nach Anspruch 10,
**dadurch gekennzeichnet, dass**
die Strickmaschine zeitgleich und parallel zur Herstellung des flexiblen Flächengebildes (50) einen ebenfalls als Flachgestrick (30) ausgebildeten Handhabungsabschnitt (60) herstetlt, wobei freie Enden der Außenabschnitte (42a) der Kompressionsfäden (42) in dieses Flachgestrick (30) des Handhabungsabschnitts (60) einelegt und dadurch fixiert werden.

## Claims

1. A compression bandage (10) for surrounding a body member, comprising
- a tubular flexible fabric (50) for surrounding the body member, wherein this fabric (50) constitutes a main body (50) which may be stretched in a circumferential direction, and
- a plurality of elastic compression threads (42) which extend in the circumferential direction guided in the plane of the fabric (50) through the fabric and achieve the compressive effect in the applied condition of the compression bandage (10),
**characterised in that**
at least some of the compression threads (42) protrude at least in portions from the fabric (50), so that they assure an adjustability of the compressive effect by varying the lengths of the respectively protruding external portions.

2. The compression bandage (10) according to Claim 1,
**characterised in that**
the tubular flexible fabric (50) is created by joining opposite edge regions (20a, 20b) of a fabric (20).

3. The compression bandage (10) according to Claim 2,
**characterised in that**
the compression threads (42) are at least partially part of a common thread (40) which runs several times to a fro between the edge regions (20a, 20b) of the fabric.

4. The compression bandage (10) according to Claim 2 or 3,
**characterised in that**
exit locations (44) where the external portions of the compression threads (42) protrude from the fabric are spaced from the associated edge, preferably by at least 5 mm, in particular preferably by at least 10 mm.

5. The compression bandage (10) according to one of the previous claims, **characterised in that**
a plurality of the elastic compression threads (42) which protrude from the fabric (50) are joined to a common handling section (60) by means of which the compression threads (42) may be pulled out of the fabric (50).

6. The compression bandage (10) according to Claim 5,
**characterised in that**
a plurality of handling sections is provided, each of which is joined to a plurality of compression threads, so that by means of the different handling sections a desired compression condition may be set individually in respective partial areas of the compression bandage.

7. The compression bandage (10) according to Claim 5 or 6,
**characterised in that**
the fabric (50) is formed as a textile fabric whose stitches guide the compression threads (42), wherein the handling section (60) is also preferably formed as a textile section which is connected to the fabric (50) only by the compression threads (42), and whose stitches also guide the compression threads (42).

8. The compression bandage (10) according to one of Claims 5 to 7,
**characterised in that**
the handling section (60) may be fixed or attached to an outer side of the main body (50) by an attachment means (70), wherein the attachment means (70) is preferably designed in such a manner that it cannot be released by a patient without using tools, and/or wherein the attachment means (70) preferable comprises
- a seam (70),
- a rivet fastening,
- a welded connection or
- an adhesive connection.

9. The compression bandage (10) according to one of the previous claims,
**characterised in that**
the compression bandage is formed as
- an arm bandage,
- a leg bandage (10), or
- a compressions stocking.

10. A method for the manufacture of a compression bandage (10) according to one of the previous claims,
**characterised in that**
1. the flexible fabric (50) is manufactured as a knitted fabric (20) by a knitting machine, wherein the compression threads (42) are introduced into the knitted fabric during the knitting process, and wherein an external portion (42a) of at least some of the compression threads (42) is led out of the flexible fabric (20) during the knitting process, and
2. two opposite edges (20a, 20b) of the knitted fabric (20) are joined in order to form the tubular main body (50).

11. The method according to Claim 10,
**characterised in that**
the knitting machine simultaneously and parallel to the manufacture of the flexible fabric (50) produces a handling section (60) which is also formed as a knitted fabric (30), wherein free ends of the external portions (42a) of the compression threads (42) are laid into said knitted fabric (30) of the handling section (60) and are thereby fixed.

## Revendications

1. Bandage de compression (10) destiné à entourer un membre du corps, comprenant
- une structure de surface (50) tubulaire souple destinée à entourer ledit membre du corps, cette structure de surface (50) formant un corps principal (50) qui peut être étiré dans une direction circonférentielle, et
- une pluralité de fils de compression (42) élastiques qui s'étendent de manière guidée à travers ladite structure de surface (50), dans le plan de cette dernière et dans une direction circonférentielle, et permettent à la structure de surface de produire son effet de compression une fois le bandage de compression (10) mis en place,
**caractérisé en ce qu'**au moins quelques-uns des fils de compression (42) dépassent au moins par endroits de la structure de surface (50) de manière à pouvoir ajuster l'effet de compression en faisant varier la longueur de la partie extérieure (42a) des fils dépassants.

2. Bandage de compression (10) selon la revendication 1,
**caractérisé en ce que** la structure de surface (50) tubulaire est obtenue par la jonction de zones de bord (20a, 20b) opposées d'une structure plate (20).

3. Bandage de compression (10) selon la revendication 2,
**caractérisé en ce que** les fils de compression (42) font au moins partiellement partie intégrante d'un fil (40) commun qui fait plusieurs fois le va-et-vient entre lesdites zones de bord (20a, 20b) de ladite structure plate (20).

4. Bandage de compression (10) selon la revendication 2 ou 3,
**caractérisé en ce que** les points de sortie (44) où les parties extérieures des fils de compression (42) dépassent de la structure de surface se trouvent à une certaine distance du bord associé, préférentiellement à une distance d'au moins 5 mm, plus particulièrement et préférentiellement à une distance d'au moins 10 mm.

5. Bandage de compression (10) selon l'une des revendications précédentes,
**caractérisé en ce qu'**une pluralité de fils de compression (42) dépassant de la structure de surface (50) est reliée à une section de maniement (60) commune au moyen de laquelle les fils de compression (42) peuvent être tirés en dehors de la structure de surface (50).

6. Bandage de compression (10) selon la revendication 5,
**caractérisé en ce qu'**il est prévu une pluralité de sections de maniement à laquelle est respectivement reliée une pluralité de fils de compression pour pouvoir ajuster dans différentes zones partielles du bandage de compression l'état de compression individuel souhaité à l'aide des différentes sections de maniement.

7. Bandage de compression (10) selon la revendication 5 ou 6,
**caractérisé en ce que** la structure de surface (50) est réalisée sous la forme d'une structure de surface textile par les mailles de laquelle passent les fils de compression (42), la section de maniement (60) étant elle aussi avantageusement conçue sous la forme d'une section textile qui n'est reliée à la structure de surface (50) que par les fils de compression (42) et par les mailles de laquelle passent également les fils de compression (42).

8. Bandage de compression (10) selon l'une des revendications 5 à 7,
**caractérisé en ce que** la section de maniement (60) peut être ou est fixée sur un côté extérieur du corps principal (50) à l'aide d'un moyen de fixation (70), ce moyen de fixation (70) étant de préférence conçu de sorte telle qu'il ne peut pas être détaché sans outil par le patient et/ou ce moyen de fixation (70) comportant de préférence
- une couture (70)
- une liaison par rivet,
- une liaison par soudure ou
- une liaison collée.

9. Bandage de compression (10) selon l'une des revendications précédentes,
**caractérisé en ce que** le bandage de compression est réalisé sous la forme
- d'un bandage pour bras
- d'un bandage pour jambe (10) ou
- d'un bas de compression.

10. Procédé pour fabriquer un bandage de compression (10) selon l'une des revendications précédentes,
**caractérisé en ce que**
1. la structure de surface (50) élastique est réalisée sous la forme d'un tricot plat (20) tricoté à la machine, les fils de compression (42) étant introduits dans le tricot plat pendant la passe de tricotage et une partie extérieure (42a) d'au moins une partie des fils de compression (42) étant sortie de la structure de surface (20) souple pendant la passe de tricotage, et
2. deux bords (20a, 20b) opposés du tricot plat (20) sont reliés l'un à l'autre pour former le corps principal (50) tubulaire.

11. Procédé selon la revendication 10,
**caractérisé en ce que** la machine à tricoter réalise simultanément et parallèlement à la réalisation de la structure de surface (50) souple également une section de maniement (60) réalisée sous la forme d'un tricot plat (30), les extrémités libres des parties extérieures (42a) des fils de compression (42) étant introduites et de ce fait fixées dans ledit tricot plat (30) de la section de maniement (60).
